# EUROPEAN PATENT APPLICATION

(11) **EP 0 850 654 A1**
(43) Date of publication of application: **01.07.1998**
(21) Application number: 97309898.1
(22) Date of filing: 05.12.1997
(51) Int. Cl.: A61M 25/00, A61F 2/06, A61B 5/06

(54) **Implantable device sensing catheter**

(30) Priority: 20.12.1996 US 33445 P
(71) Applicant: SCHNEIDER (USA) INC., Plymouth, Minnesota 55442 (US)
(72) Inventor: Kusleika, Richard S., Eden Prairie, Minnesota 55346 (US)
(74) Representative: Green, Mark Charles

(57) **Abstract**

A catheter having a circuit with sensor (14) that provides or communicates a signal relating the location of a predetermined portion of an elongated tube (17) or balloon (12) relative to an implantable device.

## Description

This invention relates generally to implantable device sensing catheters. Implantable devices such as stents are used in percutaneous transluminal coronary angioplasty and in other medical procedures to repair arteries and body lumens. For example, medical personnel often use a balloon catheter and stent to open a stenosis in an artery. On occasion, stents must be located and replaced due to insertion or dilatation complications.

Manufacturers may provide radiopaque markers on an implantable device to help a physician determine the location of an implantable device in a body lumen. In practice, the cardiologist positions a generally poorly visible stent with readily visible marker bands at the treatment site, deploys the stent. notes the location of the marker bands in relation to an arterial sidebranch or other anatomical landmark, withdraws the stent delivery system, advances a balloon catheter to the stent, attempts to position the balloon catheter marker bands in proper relation to the stent using the stent marker bands and anatomical landmarks as references, and dilatates the stent.

On occasion, it may be difficult for the cardiologist to determine where the catheter or balloon is positioned in relation to the stent. For example, some self-expanding stents may be difficult to locate because they may shorten when deployed and, thus, their initial relation to the anatomical landmarks is altered when deployed. Another reason stents may be difficult to locate is because parallax may occur when viewing the body lumen, balloon, and stent on a monitor.

Various catheters, balloon catheters, implantable devices, stents, and markers are commercially available and known in the art. Catheters and balloon catheters are, for example, disclosed in U.S. Patent Nos. 4,762.129; 5,120,323; 5,163,906; 5,232,445; 5,209,729; 5,306,247; 5,403,292; 5,505,699; 5,599,325; and 5,605,543. A device for detecting magnetic resonance signals is disclosed in U.S. Patent Serial No. 08/752,432 filed November 19,1996.

One attempt to solve the stent locatability problem is to provide a clad composite stent which contains a radiopaque core throughout its length. Such a device is shown in WO 94/16646. Another self-expanding stent, available from Schneider (USA) Inc of Minneapolis, Minnesota, has a radiopaque clad composite structure such as shown in U.S. Patent No. 5,630,840 to Mayer. Also, self-expanding stents can be made of a Titanium Alloy as described in United States Patent Application Serial No. 08/598,751, filed February 8, 1996.

Implantable devices are made of conductive and non-conductive materials known in the art. Self-expanding medical prostheses frequently referred to as stents are well known and commercially available. They are, for example, disclosed generally in the Wallsten U.S. Patent No. 4,655,771, the Wallsten et al. U.S. Patent No. 5,061,275 and in Hachtman et al., U.S. Patent No. 5,645,559. A bioabsorbable endoprosthesis is, for example, disclosed in J. Stinson's United States Patent Application entitled "Bioabsorbable Self-Expanding Stent", Serial No. 08/904467, filed August 1, 1997. Another bioabsorbable stent is disclosed in J. Stinson's United States Patent Application entitled "Bioabsorbable Implantable Endoprosthesis With Reservoir And Method Of Using Same", Serial No. 08/905,806, filed August 1, 1997.

A radiopaque marker is disclosed in J. Stinson's and Claude Clerc's United States Patent Application entitled "Radiopaque Markers And Methods Of Using Same", Serial No. 08/905,821, filed August 1, 1997. Another marker is disclosed in J. Stinson's United States Patent Application entitled "Bioabsorbable Marker Having Radiopaque Constituents And Method Of Using Same", Serial No. 08/904,951, filed August 1, 1997.

Although a radiopaque stent is an improvement for locatability purposes, the cardiologist must still rely on visual detection of the stent. In addition, radiopaque markers do not solve all of the problems associated with locating implantable devices.

The need for rapid and accurate stent locatability and methods to accomplish the same has particularly become more important with advances in micro-surgery, neuro-surgery, and conventional angioplasty procedures.

### Summary of the Invention

Accordingly, there is a need for catheters having implantable device sensing characteristics. The implantable device sensing catheter assists a physician in determining the location of a predetermined portion of a catheter or balloon relative to an implantable device in a body lumen.

Implantable devices are used within body vessels of humans for a variety of medical applications. Reference in this application to implantable devices is generally made to a stent, however, other devices include intravascular stents for treating stenoses, stents for maintaining openings in the urinary, biliary, tracheobronchial, esophageal, and renal tracts, and vena cava filters.

The present invention is preferably used with conductive implantable devices. Such conductive implantable devices may be partially coated or covered with various non-conductive materials. Such non-conductive implantable devices or materials may have exposed conductive areas or conductive marker constituents or portions thereon.

Overall, the implantable device sensing catheter relatively improves the physicians ability to locate and dilatate an implantable device in a body lumen. Use of the implantable device sensing catheter may advantageously result in stent dilatation rather than dilatation of adjacent healthy artery.

The signal from the sensors may be forwarded or transmitted to a microprocessor and to an output device such as a speaker or monitor to advantageously convey information to the physician. With the signal, the physician may be able to determine the location of a predetermined portion of the catheter or balloon relative to an implantable device.

In sum, the invention relates to an implantable device sensing catheter including an elongated tube having at least a portion of a circuit and including at least one sensor. The sensor is disposed on the elongated tube and provides one or more signals to or through the circuit relating the location of a predetermined portion of the elongated tube relative to an implantable device upon contact of the sensor with the implantable device. The implantable device may be a stent. The circuit may further include a conductive path which communicate with the sensors. The sensor and conductive path may be made of materials including metals, conductive paints, conductive epoxies, or conductive polymers. A balloon may be disposed on the elongated tube. The signal may include voltage, resistance, or amperage. The signal may be forwarded to a measurement device including a voltage meter, ohm meter, or amperage meter. The signal may be transmitted to an output device. The output device may be disposed on the catheter. The output device may include a monitor, speaker, computer, or display. The output device may provide a signal, digital signal, video signal, image, audio signal, or combination thereof relating the location of a predetermined portion of the catheter relative to the implantable device. The signal may be transmitted to a microprocessor and converted to an audio or visual signal. The signal may be based on resistance and the amount of resistance measured at an output device may corresponds to the position of the sensor in the implantable device. The resistance value at the output device may correspond with the amount of contact with the sensor. The relationship of resistance and contact may be linear or nonlinear. The implantable device is preferably at least partially conductive or includes one or more conductive constituents disposed thereon. The signal may be forwarded or transmitted to a relay. The relay may be adapted to provide wireless transmission to another device. The circuit and sensor may be at least partially disposed on the balloon.

The invention also relates to an implantable device sensing catheter including an elongated tube having at least one sensor disposed thereon. The sensor communicates one or more signals of information corresponding to the location of a predetermined portion of the elongated tube relative to a implantable device in a body lumen. The sensor communicating one or more signals when the sensor is not in contact with the implantable device, when the sensor makes contact with the implantable device, and when the sensor is substantially disposed in the implantable device.

The invention also relates to an implantable device sensing catheter including an elongated tube having at least one balloon disposed thereon. The balloon has at least one sensor disposed thereon. The sensor communicates one or more signals of information corresponding to the location of a predetermined portion of the balloon relative to an implantable device in a body lumen. The sensor communicates one or more signals when the sensor is not in contact with the implantable device, when the sensor makes contact with the implantable device, and when the sensor is substantially disposed in the implantable device. The sensor may communicate one or more signals to an output device and the output device may portrays substantially real-time information showing the relationship of the balloon to the implantable device. The output device may portrays substantially real-time information when the balloon is substantially disposed in the implantable device.

The invention also relates to a kit including an output device, an implantable device, and a catheter. The catheter having at least one circuit and sensor disposed thereon. The sensor provides one or more signals relating the location of a predetermined portion of the catheter relative to the implantable device. The implantable device may be a stent. The catheter may further include a balloon having the sensor disposed thereon.

The invention also relates to a method of dilatating a stent including the steps of inserting a stent into a body vessel; inserting a balloon catheter having at least one sensor circuit disposed thereon into the body vessel, the sensor circuit adapted to provide or communicate one or more signals to an output device relating the location of a predetermined portion of the balloon relative to the stent into a body vessel; interpreting the output device information at least once; and manipulating the balloon at least partially within the stent until the output device relates the desired position of the balloon catheter relative to the stent. The method may further include the step of dilatating the balloon at least once.

The invention also relates to an implantable device sensing catheter including an elongated tube having a balloon disposed thereon. The balloon has at least a portion of a circuit disposed thereon for detecting and providing electrical response signals. The circuit includes at least one sensor means disposed on a predetermined portion of the balloon and proximal ends adapted and arranged for interconnection to at least one of a relay, measurement device, microprocessor, or output device. The balloon is adapted to be inserted into a body lumen during a medical procedure using an implantable device. The sensor means is adapted and arranged for exposure to and contact with the implantable device during the medical procedure and to detect and provide one or more electrical signals through the circuit relating the location of the predetermined portion of the balloon relative to the implantable device upon contact of the sensor with the implantable device.

Still other objects and advantages of the present invention and methods of construction of the same will become readily apparent to those skilled in the art from the following detailed description, wherein only the preferred embodiments are shown and described, simply by way of illustration of the best mode contemplated of carrying out the invention. As will be realized, the invention is capable of other and different embodiments and methods of construction, and its several details are capable of modification in various obvious respects, all without departing from the invention. Accordingly, the drawing and description are to be regarded as illustrative in nature, and not as restrictive.

### Brief Description of the Drawings

FIG. 1 is an isometric view of a balloon catheter embodying the present invention approaching a stent;
FIG. 2 is an isometric view of another embodiment of the present invention approaching a stent;
FIG. 3A is a side view of another embodiment of the present invention approaching a stent;
FIG. 3B is a side view of the embodiment in Fig. 3A as the distal shoulder of the balloon contacts the stent;
FIG. 3C is a side view of the embodiment in Fig. 3A as the balloon is substantially disposed in the stent; and
FIG. 3D is a side view of the embodiment in Fig. 3A as the proximal shoulder of the balloon exits the stent.

### Detailed Description of the Invention

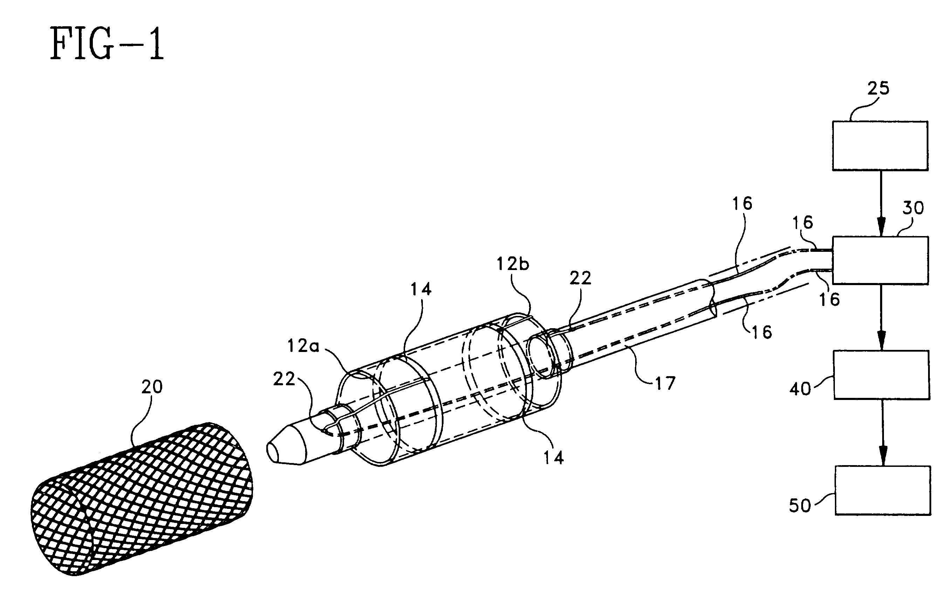

FIGS. 1-3 illustrate several embodiments of an implantable device sensing catheter **10** having a balloon **12** disposed on the shaft **17** with one or more sensors **14** disposed on the balloon **12**.

The sensing catheters **10** in FIGS. 1-3 are illustrated outside of a body lumen and in a substantially expanded state. In practice, the balloon **12** will be in a deflated state or in a minimally inflated state and inside a body lumen when the balloon **12** enters the stent **20**.

The catheter **10** may be provided with the sensors **14** positioned on the catheter **10** and without a balloon **12**. The sensors **14** may be continuous or individual separate units or circuits. For either system, a signal may occur upon contact of the sensor **14** with the stent **20** during longitudinal or radial movement of the sensor **14**, expansion of the balloon **12**, or a combination thereof. Contact between the sensor **14** and the stent **20** will generally occur at the proximal edge of the stent **20** or at the inside surface of the stent **20**.

The sensors **14** may be arranged on the catheter **10** or balloon **12** in predetermined patterns including longitudinal, circumferential, helical, curved, angular patterns, or combinations thereof. For example circumferential-shaped or U-shaped sensors **14** disposed on the balloon **12** may be used to correlate and define the position of the balloon **12** in relation to the stent **20**.

The sensors **14** may be thermally, adhesively, or chemically disposed to the catheter **10** or balloon **12**. The sensors **14** are further connected to a conductive path **16** which conducts or carries signals from the sensor **14** to a connection **22** and then to a relay **25**, measurement device **30**, microprocessor **40**, or output device **50**.

Typically, the sensors **14** are disposed on the balloon **12** surface and connected to a conductive path **16**. The conductive path **16** is connected to a connection **22** which runs through a wall **19** in the catheter shaft **17**. The connection **22** is further connected, for example, to a wire or conductive path **16** which runs along the catheter shaft **17** to the relay **25**, measurement device **30**, microprocessor **40** or output device **50**. One or more epoxy connections **22** including an epoxy-filled hole may be incorporated in the shaft **17** and circuit to make the electrical or conductive connections between the components of the system.

The sensors **14**, conductive path **16**, and connection **22** may be made of a conductive wire, conductive paint, conductive polymer, vapor deposited metal, conductive coatings, conductive film, conductive epoxy, conductive metals, or other comparable conductive materials known in the art adaptable to carry an electrical signal. The sensors **14** and conductive path **16** may be made of the same material and disposed on the balloon **12** or catheter **10** using the same methods. The sensors **14** may be made of a material having greater resistance than the conductive path **16**. For example, the shaft wires may be made of a low resistance wires such as copper and the sensor **14** may be made of conductive paint which has much more resistance than the shaft wires. The conductive path **16** may be conductive paint or conductive polymer which extends down the length of the catheter shaft **17**. The sensor **14** may have a resistance in the range of about 50 Ω (ohms) to about 2 KΩ (ohms). The sensor **14** or conductive path **16** may be made of materials having a resistance greater than 500Ω (ohms) and up to 200 KΩ (kilo-ohms).

The conductive path **16** may connect to a measurement device **30** such as an ohmmeter, voltage meter, or amperage meter. The ohmmeter may be connected to a microprocessor **40** that converts the signal to an audio signal, a video signal or image. The sensor circuit may include a microprocessor **40** programmed to output a signal to an output device **50**. The output device **50** may be any type of messaging medium including monitor, meter, speaker, indicator, imaging system, or display that provides an image, or some quantifiable information or measurement. The measurement device **30** may be the output device **50**. An output device **50** may be disposed on the proximal end of the catheter **10** to provide a reading of the balloon **12** / stent **20** relative locations. For example, a movable indicator signifying the balloon **12** may be used in a fixed dial having an image of the stent **20**. As the balloon **12** is disposed in the stent **20**, the output device **50** shows their relative relationship and, accordingly, is advantageously understandable in most any language.

A relay **25** may be incorporated in the circuit to forward the signal to at least one of the measurement device **30**, microprocessor device **40**, or output device **50**. The relay **25** may be adapted to make a wireless transmission of the signal. The microprocessor **40** may be programmed to calculate the location of the sensor **14** and balloon **12** relative to the stent **20** or to a particular region within the stent **20**.

Typically, the signals from the sensors **14** are based on resistance, voltage or amperage. Signals may be forwarded from the measurement device **30** to a microprocessor **40** for further signal processing and thereafter forwarded to an output device **50**. Signals may be forwarded to the microprocessor **40** prior to transmission to the devices **30, 50**.

The signal may be, for example, an on-off or variable signal depending on the amount of contact between the sensor **14** and the stent **20**. The signal may be dependent on the area of contact or a function of resistance of the sensor **14** with the stent **20**. The sensors **14** may have various longitudinal lengths, various areas, and resistance in order to provide a variety of signals to a user.

The conductive path **16** may be thermally, adhesively, or chemically connected to the balloon **12** or catheter **10** and, for example, be soldered to the relay **25** which may be disposed on or in a portion of the catheter **10**. Alternatively, the relay **25** may be external to the catheter **10**. One or more conductive paths **16** may be extruded in the catheter wall **19** or may run as a bundle in a lumen, inflation lumen, or channel inside or outside the catheter shaft **17**. The sensor **14** or conductive path **16**, for example, may be made of a low resistance material such as copper.

In use, a first signal may be produced and forwarded to the measurement device **30**, microprocessor **40**, or output device **50** when the balloon **14** is away from the stent **20**. One or more additional signals may be produced when the balloon **12** is at least partially disposed in the stent **20**, or upon contact between the stent **20** and the sensors **14**. The frequency of signals is dependent on the needs of the user.

In Fig. 1, two sensors **14** are arranged and disposed in predetermined patterns, for example, generally parallel and circumferential positions, around a balloon **12**. The sensors **14** are preferably located at the shoulders of the balloon **12**, but many variations are possible. In use, the sensor **14** may communicate a first signal to an output device **50** which in turn provides, for example, a first reading of zero corresponding to no contact between the sensors **14** and the stent **20**; a second signal and reading may correspond to contact of the distal most sensor **14** on the balloon **12** and the stent **20**; and a third signal and reading may correspond to contact of both sensors **14** and the stent **20**, i.e., both sensors **14** are at least partially disposed inside the stent **20** and contact has been made with the inside surface **21** of the stent **20**. Overall, a variety of signals from the output device **50** may advantageously be generated for the user.

In practice, the balloon **12** may be directed distally in the body lumen to the deployed stent **20**, and partially inflated to a low pressure. A signal or reading at the measurement device **30** or output device **50** may indicate that the balloon **12** is at least partially disposed within the stent **20**. The signal may be an audio or visual signal communicating the relation of the catheter **10** or balloon **14** to the stent **20**. The physician will determine when the balloon **12** and stent **20** are in the desired relationship.

In use, the stent **20** may short out one or more of the sensors **14** upon contact with the conductive component of the implantable device and communicate a corresponding signal to the measurement device **30**, microprocessor **40**, or output device **50**.

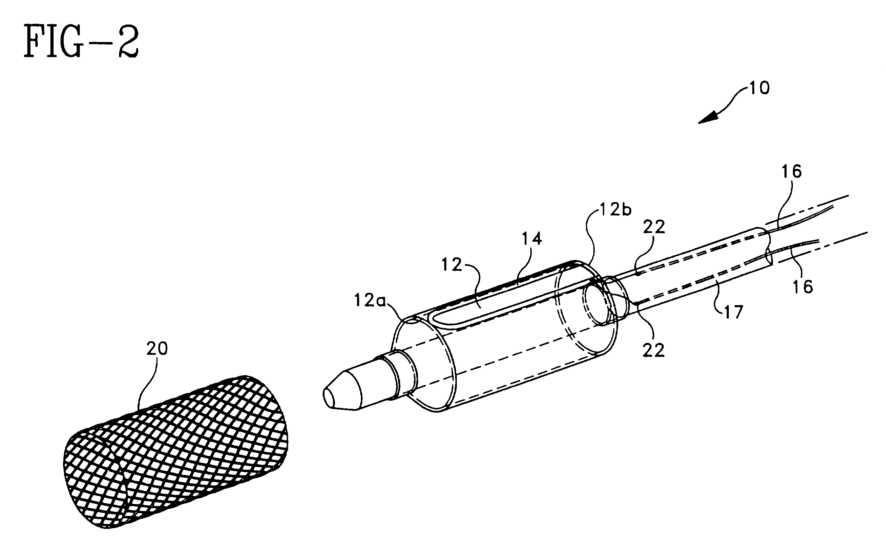

FIG. 2 illustrates a catheter **10** with a U-shaped sensors **14** disposed on the balloon **12**. In use, when the stent **20** is outside the balloon **12**, the circumferential loops of the sensor **14** may have a full resistance of, for example, a value 2X. When the stent **20** is over the distal end **12a** or proximal end **12b** of the balloon **12** and contact is made between the sensors **14** and the stent **20**, the stent 20 wires may short out the distal loop of the sensor **14** and the sensor **14** may have a resistance of, for example, a value X. Similarly, when the stent **20** is over the proximal end **12b** of the balloon **12**, the stent **20** wires may short out the proximal loop of the sensor **14** and the sensor **14** may have a resistance of, for example, a value X. When the stent **20** is substantially disposed over the balloon **12** and preferably centered, the stent **20** shorts out both loops of the sensors **14** and the sensor **14** may have a resistance of, for example, a value zero. The resistance measurements at the output device **50** would correspond or correlate to an equivalent location of the balloon **12** in relation to the stent **20**.

The resistance value and balloon **12** / stent **20** location relationship may be linear. For example, for a single longitudinal sensor **14** disposed on the balloon, the further the balloon **12** is disposed in the stent **20** and makes contact with the stent **20**, the sensor **14** has a resistance that is correspondingly lower.

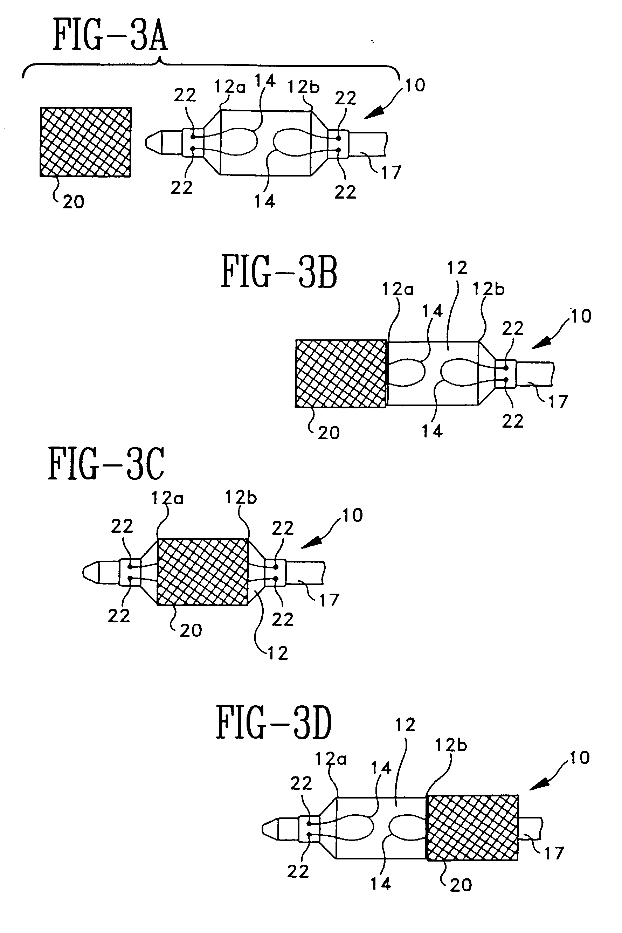

Reference is made to FIGS. 3A-D which illustrate use of the sensing catheter **10**, specifically, progressive insertion of the distal portion **12a** of the balloon **12** having one or more sensors **14** entering into the interior region of a stent **20**. Typically, as the balloon **14** approaches the stent **20**, a corresponding output from the measurement device **30**, such as an ohmmeter, relates the position of the balloon **12** in relation to the stent **20**.

The sensors **14** may be arranged such that the ohmmeter may display a high resistance or first signal when the stent **20** is not overlapping the balloon **12**; a medium resistance or second signal when the stent **20** is partially overlapping the balloon **12**; and a low resistance or third signal when the stent **20** is fully overlapping the balloon **12**.

In another embodiment, a kit for locating implantable devices may include an implantable device sensing catheter **10** having a balloon **12** with sensors **14**, and an output device **50**.

A preferred method of dilatating a stent **20** includes the steps of inserting a stent **20** into a body vessel, inserting a catheter **10** having a balloon **12** and at least one sensor **14** adapted to produce one or more signals and forward the signals to a device **30, 50** for relating the location of a predetermined portion of the balloon **12** relative to the stent **20**, interpreting the output device **50** information at least once, manipulating the balloon **12** at least partially within the stent until the device **30, 50** relates the desired position of the balloon **12** relative to the stent **20**, and dilatating the balloon **12** at least once. The method for locating a stent **20** may include steps of watching a visual display or listening for an audible signal which informs the cardiologist as to when the balloon **12** is properly positioned. The method of positioning the balloon **12** may include partial inflation of the balloon **12** or positioning the balloon **12** while deflated.

Another variation of the above method may include the step of interpreting the output device information at least once until the output device indicates that the dilatation balloon **12** is at least partially disposed or substantially disposed in the stent **20**. Also, one of the above steps may include use of a microprocessor **40** to calculate an audio or visual signal for the monitor or speaker.

It will be evident from considerations of the foregoing that the implantable device sensing may be constructed using a number of methods and materials, in a wide variety of sizes and styles for the greater efficiency and convenience of a user.

The above described embodiments of the invention are merely descriptive of its principles and are not to be considered limiting.

## Claims

1. An implantable device sensing catheter comprising:
an elongated tube (17) having at least a portion of a circuit and including at least one sensor (14), the sensor (14) disposed on the elongated tube (17) and adapted to provide one or more signals through the circuit relating the location of a predetermined portion of the elongated tube (17) relative to an implantable device upon contact of the sensor (14) with the implantable device.

2. The implantable device sensing catheter of claim 1 wherein the implantable device is a stent.

3. The implantable device sensing catheter of claim 1 or claim 2 wherein the circuit comprises a conductive path (16) which communicates with the sensors (14).

4. The implantable device sensing catheter of claim 3 wherein the sensor (14) and conductive path (16) are made of materials comprising metals, conductive paints, conductive epoxies, or conductive polymers.

5. The implantable device sensing catheter of any preceding claim wherein the signal comprises voltage, resistance, or amperage.

6. The implantable device sensing catheter of any preceding claim wherein the signal is forwarded to a measurement device (30) selected from the group consisting of a voltage meter, ohm meter, and amperage meter.

7. The implantable device sensing catheter of any preceding claim wherein the signal is transmitted to an output device (50).

8. The implantable device sensing catheter of any preceding claim wherein the signal is transmitted to a microprocessor (40) and converted to an audio or visual signal.

9. The implantable device sensing catheter of any proceeding claim wherein the signal is based on resistance and wherein the amount of resistance measured at an output device corresponds to the position of the sensor (14) in the implantable device.

10. The implantable device sensing catheter of any preceding claim wherein the signal is forwarded or transmitted to a relay (25).

11. An implantable device sensing catheter comprising:
an elongated tube (17) having at least one balloon (12) disposed thereon, the sensor (14) having at least one sensor (14) disposed thereon, the sensor (14) adapted to detect and communicate one or more signals of information corresponding to the location of a predetermined portion of the balloon (12) relative to an implantable device in a body lumen wherein the sensor (14) communicates one or more signals when the sensor (14) is not in contact with the implantable device, when the sensor (14) makes contact with the implantable device, and when the sensor (14) is substantially disposed in the implantable device.

12. An implantable device sensing catheter comprising:
an elongated tube (17) comprising a balloon (12) disposed thereon, the balloon (12) having at least a portion of a circuit disposed thereon for detecting and providing electrical response signals, the circuit including at least one sensor means disposed on a predetermined portion of the balloon (12) and proximal ends adapted and arranged for interconnection to at least one of a relay (25), measurement device (30), microprocessor (40), or output device (50), the balloon (12) adapted to be inserted into a body lumen during a medical procedure using an implantable device wherein the sensor means is adapted and arranged for exposure to and contact with the implantable device during the medical procedure and to detect and provide or more electrical signals through the circuit relating the location of the predetermined portion of the balloon (12) relative to the implantable device upon contact of the sensor with the implantable device.
